# EUROPÄISCHE PATENTSCHRIFT

(11) **EP 3 546 002 B1**
(45) Veröffentlichungstag und Bekanntmachung des Hinweises auf die Patenterteilung: **17.02.2021**
(21) Anmeldenummer: 19165059.7
(22) Anmeldetag: 26.03.2019
(51) Int. Cl.: A61M 16/04, G06T 17/00, A61M 16/06, A61F 2/20

(54) **TRACHEOSTOMAEPITHESE, EPITHESEN-GUSSFORM UND VERFAHREN ZUR HERSTELLUNG EINER TRACHEOSTOMAEPITHESE**
TRACHEOSTOMA EPITHESIS, EPITHESIS MOULD AND METHOD FOR PRODUCING A TRACHEOSTOMA EPITHESIS
TRACHÉOSTOMIE-ÉPITHÈSE, LINGOTIÈRE D'ÉPITHÈSE ET PROCÉDÉ DE FABRICATION D'UNE TRACHÉOSTOMIE-ÉPITHÈSE

(30) Priorität: 29.03.2018 DE 102018107680
(43) Veröffentlichungstag der Anmeldung: 02.10.2019
(73) Patentinhaber: Andreas Fahl Medizintechnik-Vertrieb GmbH, 51149 Köln (DE)
(72) Erfinder: FAHL, Andreas, 51107 Köln (DE)
(74) Vertreter: Geskes, Christoph

(56) Entgegenhaltungen:
- EP-A2- 2 584 534
- WO-A1-2018/009118
- DE-U1- 20 303 669
- DE-U1-202006 008 753
- DE-U1-202015 007 408
- BOZZATO V ET AL: "Satisfaction of patients with tracheostomal epithesis", HNO. HALS-, NASEN-, OHRENAERZTE, SPRINGER VERLAG, BERLIN, DE, Bd. 64, Nr. 4, 29. März 2016 (2016-03-29), Seiten 243-253, XP035721615, ISSN: 0017-6192, DOI: 10.1007/S00106-016-0148-4 [gefunden am 2016-03-29]
- BOZZATO V ET AL: "Epithetic replacement in otorhinolaryngology", HNO. HALS-, NASEN-, OHRENAERZTE, SPRINGER VERLAG, BERLIN, DE, Bd. 63, Nr. 10, 20. August 2015 (2015-08-20), Seiten 727-738, XP035551903, ISSN: 0017-6192, DOI: 10.1007/S00106-015-0035-4 [gefunden am 2015-08-20]

## Beschreibung

Die Erfindung betrifft eine Tracheostomaepithese, eine Epithesen-Gussform zur Herstellung einer Tracheostomaepithese sowie ein Verfahren zur Herstellung einer Tracheostomaepithese.

Aus dem Stand der Technik sind Epithesen zum ästhetischen Ausgleich von Körperdefekten mittels eines körperfremden Materials wie beispielsweise Glas, Porzellan, Gummi, Metall oder Kunststoff hinlänglich bekannt. Epithesen werden zumeist mittels eines Gipsabdrucks an die defekte Stelle des Körpers angepasst und von Hand nachgestaltet.

Die DE 2011116 386 A1 beschreibt ein medizinisches, therapeutisches oder kosmetisches Produkt, das unter Nutzung eines 3D-Scans herstellbar ist, wobei der 3D-Scan in ein Voxel-Format konvertiert wird und mittels einer Formmaschine anhand der Voxel-Daten das Produkt hergestellt wird.

WO 2018/009118 A1 offenbart ein Tracheostomapflaster mit einer rigiden Platte und einem Tracheostomavorrichtungshalter, der distal auf der Platte angeordnet ist.

Dr. med. V. Bozzato et. al (2015), Epithetische Versorgung in der HNO-Heilkunde, Springer-Verlag Berlin Heidelberg offenbart eine Tracheostomaepithese zur funktionellen Rehabilitation.

Dr. med. V. Bozzato et. al (2016), Die Tracheostomaepithese - eine Evaluation der Patientenzufiedenheit, Springer-Verlag Berlin Heidelberg offenbart eine Herstellung einer Tracheostomaepithese mittels einer Gussform.

EP 2 584 534 A2 offenbart ein computerimplementiertes Verfahren zum Erzeugen eines virtuellen 3D-Modells eines realen dreidimensionalen Objektes sowie ein auf dieser Grundlage geformtes Produkt.

Insbesondere für Patienten mit einem Tracheostoma ist die übliche Epithesenherstellung unangenehm. In der Regel wird ein Gipsabdruck der defekten Stelle genommen um die Epithese an diese anzupassen. In jedem Fall besteht bei tracheotomierten Patienten die Gefahr der Respiration des Abdruckmittels und Atemnot während der Zeit des Abdrucks. Weiterhin ist der Nachteil der bekannten Epithesen für Tracheotomierte seine Luftführung nicht optimal erfolgt und insbesondere ein Tracheostomapflaster nur unzureichend auf der Epithese befestigbar ist.

Aufgabe der Erfindung ist es daher, eine verbesserte Tracheostomaepithese, die insbesondere die aus dem Stand der Technik bekannten Nachteile überkommt, zur Verfügung zu stellen. Weiterhin ist es Aufgabe der Erfindung eine verbesserte Epithesen-Gussform sowie ein verbessertes Verfahren zur Herstellung einer Tracheostomaepithese zur Verfügung zu stellen. Die Aufgabe wird erfindungsgemäß gelöst mittels einer Tracheostomaepithese umfassend einen Epithesenkörper mit einer proximalen Seite, einer distalen Seite und einer vollständig von proximal nach distal durchgehenden Ausnehmung, wobei die Ausnehmung im Wesentlichen konisch ist und sich nach distal verjüngt.

Die Aufgabe wird weiterhin erfindungsgemäß gelöst mittels einer Epithesen-Gussform zur Herstellung eine Tracheostomaepithese zumindest umfassend eine Kavität, wobei diese einen konischen Kern umfasst, wobei der konische Kern derart in der Kavität angeordnet ist, dass eine Grundfläche des Konus einem proximalen Teil der Gussform zugeordnet ist, der die proximale Seite der Tracheostomaepithese formt.

Die Aufgabe wird des Weiteren erfindungsgemäß gelöst mittels eines Verfahrens zur Herstellung einer Tracheostomaepithese, umfassend die Schritte
- Bereitstellen einer Epithesen-Gussform,
- Einbringen eines konischen Kerns in die Epithesen-Gussform,
- Gießen eines Epithesenmaterials in die Epithesen-Gussform,

Es wird eine Tracheostomaepithese umfassend einen Epithesenkörper vorgeschlagen. Die Tracheostomaepithese weist eine proximale Seite, eine distale Seite und eine vollständig von proximal nach distal durchgehenden Ausnehmung auf, wobei die Ausnehmung im Wesentlichen konisch ist und sich nach distal verjüngt.

Wenn im Rahmen der Beschreibung Richtungsangaben verwendet werden, so sind diese im Bezug auf den üblichen Gebrauch der Tracheostomaepithese zu verstehen. Richtungsbezeichnungen werden wie in der Anatomie üblich verwendet. Unter dem Begriff "distal" wird im Sinne der vorliegenden Erfindung eine Anordnung beziehungsweise Verwendung eines Merkmals fern oder auch abgewandt einer Hautoberfläche eines Benutzers, der insbesondere die Tracheostomaepithese trägt, verstanden. Unter "distal" ist vorzugsweise eine Richtung vom Patienten weg zu verstehen. Unter "proximal" wird im Sinne der vorliegenden Erfindung eine Anordnung beziehungsweise Verwendung eines Merkmals nah oder auch zugewandt einer Hautoberfläche des Benutzers, der insbesondere die Tracheostomaepithese trägt, verstanden. Unter "proximal" ist vorzugsweise eine Richtung zum Patienten hin zu verstehen.

Der Begriff "im Wesentlichen" gibt einen Toleranzbereich an, der für den Fachmann unter wirtschaftlichen und technischen Gesichtspunkten zu vertreten ist, sodass das entsprechende Merkmal noch als solches zu erkennen oder verwirklicht ist.

Bei einer Tracheotomie oder Laryngotomie gelingt es dem Chirurgen nicht immer ein rundes gut passendes Tracheostoma insbesondere in einem Halsprofil anzulegen, welches um das Tracheostoma relativ flach ist. Zudem können postoperative Heilungsstörungen und Tumorrezidive die Tracheostomaform ungünstig beeinflussen. Gegebenenfalls kann es dazu kommen, dass Kanülen nicht in das Tracheostoma passen und/oder Pflaster nicht auf den umgebenden Hautbereich des Tracheostomas haften. In diesen Fällen wird eine Tracheostomaepithese für den Patienten angefertigt. Insbesondere wenn die Haut um das Tracheostoma faltig ist und/oder Nischen aufweist und/oder das Tracheostoma ungünstig zwischen den Ansätzen des Musculus sternocleidomastoideus liegt, ist das luftdichte Abdichten zum Sprechen mit Standardhilfsmittel nicht zu erreichen. Zur Herstellung der Tracheostomaepithese sind individuelle Abformmittel, wie beispielsweise Gips oder eine Silikonabformmasse, bekannt. In einigen Fällen wird in die Epithese eine Standardkanüle integriert oder die Epithese erhält einen Standardkonnektor für Tracheostomahilfsmittel. Die aus dem Stand der Technik bekannten Epithesen weisen in der Regel eine Formgebung auf, die zumindest teilweise in die Trachea eindringt und insbesondere das Tracheostoma verengt.

In einer Ausgestaltung ist vorgehen, dass die Tracheostomaepithese zumindest ein Epithesenmaterial aufweist ausgewählt aus einer Gruppe umfassend Silikonkautschuk, Silikonelastomere und/oder thermoplastische Elastomere. Weiter bevorzugt ist vorgesehen, dass die Tracheostomaepithese ein Zweikomponentenkautschuk umfasst, beziehungsweise aus diesem hergestellt ist. Weiter bevorzugt ist vorgesehen, dass der Epithesenkörper das Epithesenmaterial aufweist, weiter bevorzugt aus diesem hergestellt ist. Das Epithesenmaterial ist insbesondere ein gießfähiges Material, das bei etwa 15°C bis etwa 80°C aushärtet. Insbesondere ist vorgesehen, dass das Epithesenmaterial ein Zweikomponentenkautschuk umfasst, der bei einer Zimmertemperatur von etwa 20°C aushärtet. Weiter bevorzugt wird eine Aushärtung des Epithesenmaterials bei einer höheren Temperatur insbesondere etwa 60°C bis etwa 80°C beschleunigt.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 %, vorgesehen.

Der Epithesenkörper ist in einer Ausgestaltung individuell an eine Defektstelle eines Benutzers angepasst, insbesondere an ein Tracheostoma sowie an den das Tracheostoma umgebenden Bereich. In einer weiteren Ausgestaltung ist vorgesehen, dass der Epithesenkörper eine vorgefertigte Formgebung aufweist. Bevorzugt verjüngt sich ein Durchmesser des Epithesenkörpers von distal nach proximal. Weiter bevorzugt weist der Epithesenkörper in einer Aufsicht von distal eine rechteckige, eine ovale, eine runde oder eine sonstige Formgebung auf. Insbesondere können Ecken abgerundet sein und/oder eine oder mehrere der vorgenannten Formgebungen zu weiteren Formgebungen miteinander kombiniert werden. Bevorzugt ist der Epithesenkörper kegelförmig ausgestaltet, wobei die distale Seite eine Grundfläche einer Kegelform und weiter bevorzugt die proximale Seite einer Mantelfläche der Kegelform entspricht. Insbesondere ist die distale Seite rund, elliptisch, rechteckig, insbesondere mit abgerundeten Kanten oder in einer anderen beliebigen Form ausgebildet. In einer Ausgestaltung ist vorgesehen, dass der Epithesenkörper auf der distalen Seite eine distale Oberfläche aufweist, die insbesondere im Wesentlichen eben ausgestaltet ist. In einer Ausgestaltung ist vorgesehen, dass der Epithesenkörper auf der proximalen Seite eine proximale Oberfläche aufweist, die bevorzugt im Wesentlichen kegelmantelförmig und/oder im Wesentlichen eine Negativform eines ein Tracheostoma umgebenen Hautbereiches ist. Weiterhin ist bevorzugt vorgesehen, dass eine Hochachse des Kegels beziehungsweise des Epithesenkörper mit einer Längsachse der Ausnehmung übereinstimmt.

Im Sinne der Erfindung ist ein Kegel beziehungsweise ein Konus ein geometrischer Körper, bei dem alle Punkte einer Grundfläche gradlinig mit einer Spitze beziehungsweise einem Punkt einer Ebene, in der nicht die Grundfläche angeordnet ist, verbunden sind. Die Grundfläche kann kreisförmig, rechteckig, oval oder jede beliebige andere Form, insbesondere mit abgerundeten Ecken, aufweisen. Weiterhin weist im Sinne der Erfindung eine konische Ausnehmung bevorzugt eine kreiskegelförmige Ausgestaltung auf. Unter einem Kegel oder einem Konus kann im Sinne der Erfindung auch ein Kegelstumpf verstanden werden. Insbesondere ist vorgesehen, dass die konische Ausnehmung sich derart durch den kegelförmigen Epithesenkörper erstreckt, dass der Epithesenkörper als Kegelstumpf ausgebildet ist. Weiterhin weist bevorzugt die konische Ausnehmung eine theoretische Konusspitze auf, die außerhalb des Epithesenkörpers liegt. Somit ist eine Ausnehmungswandungsoberfläche, die die Ausnehmung innerhalb des Epithesenkörpers begrenzt, kegelstumpfförmig beziehungsweise weist die Form eine Mantels eines Konusstumpfes auf. In einer Ausgestaltung ist eine Längsachse der Ausnehmung vorzugsweise mit der Hochachse des kegelförmigen Epithesenkörpers, die ein Lot von einer Spitze auf die Grundfläche der Kegelform ist, kongruent.

Die Ausnehmung ist im Wesentlichen konisch ausgestaltet und verjüngt sich nach distal. Die Ausnehmung weist insbesondere eine in einem Querschnitt kreisförmige Ausgestaltung auf. Eine proximale Öffnung bildet die Grundfläche des Konus. Weiter bevorzugt entspricht eine distale Öffnung einer Deckfläche des stumpfförmigen Konus, der die Ausnehmung formt. Die Ausnehmung wird insbesondere durch die Ausnehmungswandungsoberfläche, die sich von proximal nach distal erstreckt und deren Umfang sich nach distal verringert, begrenzt. Die Ausnehmungswandungsoberfläche weist bevorzugt eine Form eines Konusmantels, bevorzugt eines Konusstumpfes, auf. Weiterhin bevorzugt ist die Ausnehmungswandungsoberfläche im Herstellungsprozess mittels eines konischen Kerns geformt. Weiter bevorzugt grenzt die Ausnehmungswandungsoberfläche distal an die distale Oberfläche des Epithesenkörpers. Weiter bevorzugt grenzt die Ausnehmungswandungsoberfläche proximal an die proximale Oberfläche des Epithesenkörpers.

Ein Öffnungswinkel des Konus bestimmt sich insbesondere durch einen Durchmesser der distalen Öffnung und einen Durchmesser der proximalen Öffnung sowie der Dicke der Tracheostomaepithese beziehungsweise des Epithesenkörpers. Bevorzugt ist der Durchmesser der distalen Öffnung etwa 5 mm bis etwa 22 mm, weiter bevorzugt etwa 10 mm bis etwa 22 mm, weiter bevorzugt etwa 15 mm. In einer Ausgestaltung ist der Durchmesser der proximalen Öffnung etwa 22 mm bis etwa 40 mm, bevorzugt etwa 22 mm bis etwa 30 mm, weiter bevorzugt etwa 22 mm. Eine Höhe der Tracheostomaepithese von proximal nach distal ist in einer Ausgestaltung etwa 2 mm bis etwa 50 mm, bevorzugt etwa 2 mm bis etwa 25 mm, weiter bevorzugt etwa 8 mm bis etwa 25 mm.

Durch diese vorgeschlagene Ausgestaltung wird weiterhin auch bei vorkonfektionierten Tracheostomaepithesen eine optimale Anpassung an das Tracheostoma und den umliegenden Hautbereich ermöglicht. Weiterhin ist ein Atemstrom, der durch die Ausnehmung geführt wird, optimal für Tracheostomahilfsmittel kanalisiert. Die distale Öffnung der Ausnehmung ist insbesondere derart gestaltet, dass ein Tracheostmapflaster auf der Tracheostomaepithese und gegebenenfalls dem umliegenden Gewebe aufgebracht werden kann, so dass die distale Öffnung der Tracheostomaepithese mit der proximalen Öffnung des Tracheostomapflasters deckungsgleich oder kleiner als diese ist. Die Ausgestaltung des Konus der Ausnehmung hat den großen Vorteil, insbesondere bei Verwendung eines Tracheostomapflasters, dass die Atemluft, insbesondere beim Ausatmen, ausschließlich durch die im Tracheostomapflaster vorgesehene Ausnehmung geleitet wird. Hierdurch wird vermieden, dass ein Luftpolster unter dem Tracheostomapflaster gebildet wird, welches sich gegebenenfalls beim Sprechen mit einem Sprechventil aufbläst und zur Ablösung des Tracheostomapflasters führen kann. Insbesondere ist daher vorgesehen, dass die distale Öffnung kleiner ist als die Öffnung des Tracheostomapflasters, die etwa 20 mm bis etwa 22 mm betragen kann.

In einer weiteren Ausführungsform ist vorgesehen, dass die proximale Seite des Epithesenkörpers an die Formgebung eines Tracheostomas und dessen umliegenden Gewebes eines Benutzers angepasst ist. Weiter bevorzugt weist auch die angepasste Ausgestaltung eine konische Ausnehmung auf, dessen Form sich nach distal verjüngt.

Eine erste beispielhafte Tracheostomaepithese weist einen Epithesenkörper mit einer distalen und einer proximalen Seite auf. Die proximale Seite weist eine proximale Oberfläche des Epithesenkörpers auf, die in Form eines Kegelmantels ausgestaltet ist. Die distale Seite weist eine distale Oberfläche des Epithesenkörpers auf, die im Wesentlichen eben ausgestaltet ist und eine Kegelgrundfläche formt. Eine beispielhafte Höhe des Epithesenkörpers von proximal nach distal ist etwa 8 mm bis etwa 25 mm, wobei die Tracheostomaepithese insbesondere in einer oder mehreren vorgegebenen Höhen vorkonfektioniert ist.

Eine zweite beispielhafte Tracheostomaepithese weist einen Epithesenkörper mit einer distalen und einer proximalen Seite auf. Die proximale Seite weist eine proximale Oberfläche des Epithesenkörpers auf, die im Wesentlichen einer Form eines ein Tracheostoma umgebenen Hautbereiches nachgeformt ist. Die distale Seite weist eine distale Oberfläche des Epithesenkörpers auf, die ist im Wesentlichen eben ausgestaltet ist und bevorzugt eine Hautoberfläche nachformt. Eine beispielhafte Höhe des Epithesenkörpers von proximal nach distal ist etwa 8 mm bis etwa 25 mm, wobei die Höhe der Tracheostomaepithese insbesondere an ein Tracheostoma eines Benutzers angepasst ist.

In beiden beispielhaften Ausgestaltungen, aber nicht nur in diesen, ist vorgesehen, dass vollständig von proximal nach distal sich eine Ausnehmung durch den Epithesenkörper erstreckt, die von einer Ausnehmungswandungsoberfläche begrenzt ist. Die Ausnehmung verjüngt sich im Wesentlichen nach distal, wobei die Ausnehmungswandungsoberfläche bevorzugt eine Form eines Konusmantels aufweist. Die Ausnehmungswandungsoberfläche grenzt distal unmittelbar an die distale Oberfläche des Epithesenkörpers. Die Ausnehmungswandungsoberfläche grenzt weiterhin proximal unmittelbar an die proximale Oberfläche des Epithesenkörpers. Die Ausnehmung weist in den gegebenen Beispielen distal und proximal eine jeweils kreisrunde Öffnungsfläche auf. Die distale kreisrunde Öffnungsfläche hat einen beispielhaften Durchmesser von etwa 15 mm. Die proximale kreisrunde Öffnungsfläche hat einen beispielhaften Durchmesser von etwa 22 mm.

Weiterhin wird eine Epithesen-Gussform, zumindest umfassend eine Kavität und einen konischen Kern vorgeschlagen. Die Epithesen-Gussform ist insbesondere zweiteilig ausgeführt. Die Epithesen-Gussform weist zumindest einen ersten Teil auf, der insbesondere eine proximale Seite der Tracheostomaepithese formt. Weiterhin bevorzugt weist die Epithesen-Gussform einen zweiten Teil auf, der die distale Seite der Tracheostomaepithese formt. In einer weiteren Ausgestaltung ist die Tracheostomaepithese einteilig ausgestaltet. Insbesondere formt die einteilige Epithesen-Gussform die proximale Seite der Tracheostomaepithese, wobei die distale Seite der Tracheostomaepithese im Wesentlichen nicht geformt beziehungsweise eben bleibt. Weiter bevorzugt ist die einteilige Epithesen-Gussform einseitig offen ausgestaltet. Besonders bevorzugt ist vorgesehen, dass die Epithesen-Gussform derart ausgestaltet ist, dass diese eine oben beschriebene Tracheostomaepithese formt.

Die insbesondere zumindest zweiteilig gestaltete Kavität ist des Weiteren derart ausgestaltet, dass diese den konischen Kern aufnimmt. Erfindungsgemäß ist der konische Kern derart in der Kavität angeordnet, dass eine Grundfläche des Konus dem proximalen Teil der Gussform zugeordnet ist - also dem Teil der Gussform der die proximale Seite der Tracheostomaepithese formt.

In einer weiteren Ausgestaltung ist vorgesehen, dass die Epithesen-Gussform mittels eines generativen Fertigungsverfahrens oder eines Tiefziehverfahrens hergestellt ist. Vorzugsweise ist die Epithesen-Gussform mittels eines 3D-Drucks hergestellt. Dies erlaubt insbesondere die individuelle Anpassung an einen Patienten. In einer weiteren Ausführungsform ist vorgesehen, dass die Epithesen-Gussform mittels eines Tiefziehverfahrens hergestellt ist. Dies erlaubt insbesondere eine einfache und kostengünstige Herstellung von vorkonfektionierten Tracheostomaepithesen, beispielsweise in einer verlorenen Form. Insbesondere ist in einer Ausführungsform vorgesehen, dass die Epithesen-Gussform eine Mehrzahl von gleichen oder unterschiedlichen Kavitäten für die Herstellung von Tracheostomaepithesen aufweist. Beispielsweise ist die Epithesen-Gussform, insbesondere eine tiefgezogene Epithesen-Gussform, Teil einer Produktverpackung, insbesondere ein Verpackungseinleger. Eine Ausgestaltung der Epithesen-Gussform, die Teil einer Produktverpackung ist, hat den Vorteil, dass die Epithesen in der Epithesen-Gussform gegossen und unmittelbar, gegebenenfalls nach dem Aushärten des Epithesenmaterials, verpackt werden können, ohne dass diese vorher entformt werden müssen. Die Epithesen sind somit unmittelbar nach dem Gießen sicher verpackt. Eine Restaushärtung des Epithesenmaterials kann gegebenenfalls fertig verpackt in einem Produktlager erfolgen.

In einer weiteren bevorzugten Ausgestaltung ist vorgesehen, dass die Epithesen-Gussform zumindest ein Gussformmaterial, ausgesucht aus einer Gruppe von hautverträglichen Kunststoffen und/oder Photopolymeren umfasst.

Weiterhin wird eine Verwendung der oben beschriebenen Epithesen-Gussform als Verpackungseinleger vorgeschlagen.

Besonders bevorzugt ist vorgesehen, dass die Kavität der Epithesen-Gussform zumindest teilweise ein Strukturabbild einer Epithesenaufnahmeoberfläche, die ein Tracheostoma eines Patienten umgibt, aufweist. Auf diese Weise kann eine patientengerechte Anpassung der Tracheostomaepithese erfolgen. Die Epithesenaufnahmeoberfläche ist bevorzugt eine Hautoberfläche und ein das Tracheostoma umgebenes Gewebe und weiter bevorzugt ein Tracheostomarandgewebe eines Halses des Benutzers.

Weiterhin wird ein Verfahren zur Herstellung einer Tracheostomaepithese vorgeschlagen, umfassend die Schritte
- Bereitstellen einer Epithesen-Gussform,
- Einbringen eines konischen Kerns in die Epithesen-Gussform,
- Gießen eines Epithesenmaterials in die Epithesen-Gussform,

In einer Ausführungsform ist vorgesehen, dass die Tracheostomaepithese insbesondere nach einer Aushärtung des Epithesenmaterials entformt wird. In einer weiteren Ausgestaltung ist vorgesehen, dass die Tracheostomaepithese mit der Epithesen-Gussform verpackt wird. Insbesondere kann eine Mehrzahl von Tracheostomaepithesen mit einer Epithesen-Gussform, die bevorzugt eine Mehrzahl von Kavitäten aufweist, bevorzugt gleichzeitig gegossen und weiter bevorzugt danach ohne vorher entformt zu werden verpackt werden.

Insbesondere kann eine Gussform bereitgestellt werden, die vorkonfektioniert ist. Der konische Kern wird in einer Ausgestaltung in die Gussform eingesetzt. In einer weiteren Ausgestaltung ist vorgesehen, dass der konische Kern mit der Gussform gefertigt wird. Besonders bevorzugt ist vorgesehen, dass das Epithesenmaterial vor dem Eingießen in die Gussform an eine Hautfarbe des Benutzers farblich angepasst wird. Weiterhin ist in einer Ausgestaltung vorgesehen, dass bei einer Vielzahl von unterschiedlichen Epithesenmaterialien in die Gussform eingegossen wird, um insbesondere unterschiedliche Färbung und/oder unterschiedliche Härten in verschiedenen Bereichen der Tracheostomaepithese zu generieren. Insbesondere ist einer Ausführungsform vorgesehen, dass ein Epithesenmaterial mit einer höheren Shore-A-Härte um die Ausnehmung vorgesehen ist als an den äußeren Rändern der Tracheostomaepithese.

In einer weiteren Ausführungsform ist vorgesehen, dass das Bereitstellen der Gussform die Schritte umfasst
- Abscannen einer Epithesenaufnahmeoberfläche mittels eines 3D-Scanners,
- Nachbearbeiten von Scandaten und Genieren eines Strukturabbildes der Epithesenaufnahmeoberfläche und Formen einer virtuellen Epithesen-Gussform mit diesem,
- Drucken einer Gussform entsprechend der virtuellen Epithesen-Gussform mittels eines generativen Fertigungsverfahrens.

Besonders bevorzugt ist vorgesehen, dass ein 3D-Scanner, ein Laserscanner, ein Streifenlichtscanner oder eine TOF-Kamera aufweist. Auch können weitere Bildgebungsverfahren verwendet werden, die ein dreidimensionales virtuelles Abbild der Epithesenaufnahmeoberfläche anfertigen können. Diese Epithesenaufnahmeoberfläche wird mittels des 3D-Scanners gescannt und die Oberfläche virtualisiert. Die Scandaten werden in einer Ausführungsform beispielsweise als Voxel-Daten gespeichert und automatisiert und/oder manuell derart nachbearbeitet, dass ein virtuelles Strukturabbild der Epithesenaufnahmeoberfläche herstellbar ist. Insbesondere ist vorgesehen, dass beim Generieren der virtuellen Epithesen-Gussform der konische Kern unmittelbar mitgestaltet wird, so dass dieser bei der Fertigung der Gussform mittels des generativen Fertigungsverfahrens mitgefertigt werden kann. In einer weiteren Ausgestaltung wird der konische Kern nach der Fertigung der Gussform in diese eingesetzt.

In einer beispielhaften Ausgestaltung ist vorgesehen, dass für das Verfahren zur Herstellung einer Tracheostomaepithese eine vorkonfektionierte Gussform bereitgestellt wird, die einen konischen Kern aufweist oder in die ein konischer Kern eingefügt wird, wobei nach Gießen des Epithesenmaterials in die Epithesen-Gussform die Tracheostomaepithese in dieser aushärtet. Insbesondere kann die Epithesen-Gussform aus einem tiefgezogenen Material gefertigt sein und als Verpackungseinleger fungieren, so dass die Tracheostoma-Epithese vor dem Verpacken nicht entformt werden muss. Dies hat den Vorteil, dass eine große Stückzahl Tracheostomaepithesen herstellbar sind, die für viele Benutzer vollkommen ausreichen und eine zufriedenstellende Abdichtung sowie Luftführung ermöglichen. Insbesondere ist vorgesehen, dass eine Vielzahl von vorkonfektionierten Epithesen-Gussformen oder Kavitäten in einer Epithesen-Gussform vorgesehen ist, die eckig, oval, rund oder Mischformen dessen aufweisen und weiter bevorzugt eine kegelförmige proximale Manteloberfläche aufweisen, um damit eine Vielzahl von unterschiedlichen Tracheostomata beziehungsweise Epithesenaufnahmeoberflächen gerecht zu werden.

In einer zweiten beispielhaften Ausgestaltung ist vorgesehen, dass das Verfahren zur Herstellung einer Tracheostomaepithese die Schritte umfasst
- Abscannen einer Epithesenaufnahmeoberfläche mittels eines 3D-Scanners,
- das manuelle und/oder automatisierte Nacharbeiten der Scandaten, insbesondere zum Entfernen von gegebenenfalls vorhandenen virtuellen Artefakten, zur Generierung eines Strukturabbildes der Epithesenaufnahmeoberfläche,
- Erzeugen einer virtuellen Epithesen-Gussform mit diesem Strukturabbild und einsetzen eines konischen Kerns in die virtuelle Epithesen-Gussform,
- Wandeln der virtuellen Epithesen-Gussform in 3D-Druckerdaten,
- Senden der 3D-Druckerdaten an einen 3D-Drucker zur generativen Herstellung der Epithesen-Gussform, und
- Einbringen von Epithesenmaterial in die Gussform sowie bevorzugt Härten des Epithesenmaterials insbesondere in einem Ofen,
- Entformen der Tracheostomaepithese und gegebenenfalls manuelles Nacharbeiten.

In beiden genannten Ausgestaltungen ist beispielsweise die Verwendung Silikonkautschuk, weiterhin bevorzugt Zweikomponentensilikonkautschuk als Epithesenmaterial vorgesehen.

Weitere vorteilhafte Ausgestaltungen gehen aus den nachfolgenden Figuren hervor. Die dort dargestellten Weiterbildungen sind jedoch nicht beschränkend auszulegen, vielmehr können die dort beschriebenen Merkmale untereinander und mit den oben beschriebenen Merkmalen zur weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen
- Fig. 1: eine vorkonfektionierte Tracheostomaepithese;
- Fig. 2: eine weitere Ausgestaltung einer vorkonfektionierten Tracheostomaepithese;
- Fig. 3: eine weitere Ausgestaltung einer vorkonfektionierten Tracheostomaepithese;
- Fig. 4: eine weitere Ausgestaltung einer vorkonfektionierten Tracheostomaepithese;
- Fig. 5: eine weitere Ausgestaltung einer vorkonfektionierten Tracheostomaepithese;
- Fig. 6: eine weitere Ausgestaltung einer vorkonfektionierten Tracheostomaepithese;
- Fig. 7: eine weitere Ausgestaltung einer vorkonfektionierten Tracheostomaepithese; und
- Fig. 8: ein Verfahren zur Herstellung einer individuell angepassten Tracheostomaepithese.

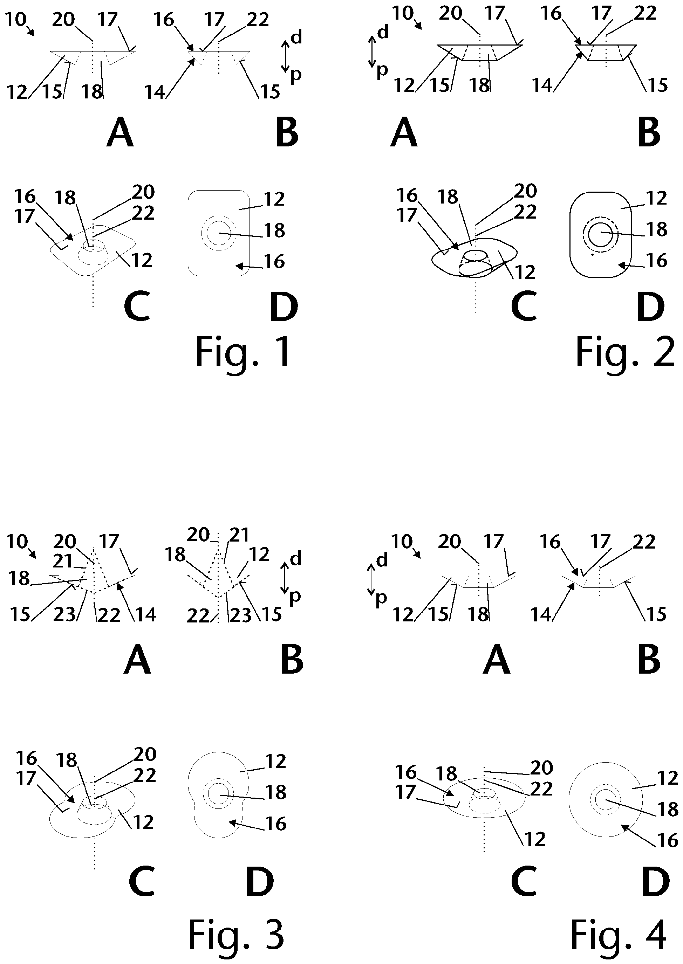

Fig. 1 bis Fig. 7 zeigt eine Auswahl von Varianten von insbesondere vorkonfektionierten Tracheostomaepithesen jeweils aus zwei Seitenansichten A und B, einer perspektivischen Ansicht C und einer Aufsicht D von distal. Die Tracheostomaepithesen 10 weisen jeweils einen Epithesenkörper 12 auf, der eine proximale Seite 14 und eine distale Seite 16 umfasst. Die distale Seite 16 weist eine distale Oberfläche 17 auf, die im Wesentlichen eben ist. Eine proximale Oberfläche 15 auf der proximalen Seite 14 des Epithesenkörpers 12 ist im Wesentlichen entsprechend einer Mantelfläche eines Kegels beziehungsweise Kegelstumpfes geformt, dessen Grundfläche der jeweiligen distalen Oberfläche 17 entspricht. Von der distalen Seite 16 zur proximalen Seite 14 erstreckt sich eine Ausnehmung 18, die konisch ausgestaltet ist, wobei die konische Ausnehmung 18 sich von proximal p nach distal d verjüngt. Insbesondere ist die distale nicht weiter bezeichnete Öffnung der Ausnehmung 18 im Wesentlichen kreisrund.

Die Längsachse 20 der Ausnehmung 18 ist vorzugsweise mit der Hochachse 22 des kegelförmigen Epithesenkörpers 12, die ein Lot von einer Spitze auf die Grundfläche der Kegelform ist, kongruent. Der Kegel 23 des Epithesenkörpers 12 ist beispielhaft in Fig. 3 angedeutet. Auch ist Fig. 3 der Konus 21 der Ausnahme 18 zu entnehmen. Der Epithesenkörper 12 ist aufgrund der Überlagerung seines Kegels 23 mit dem Konus 21 der Ausnehmung 18 als Kegelstumpf ausgebildet.

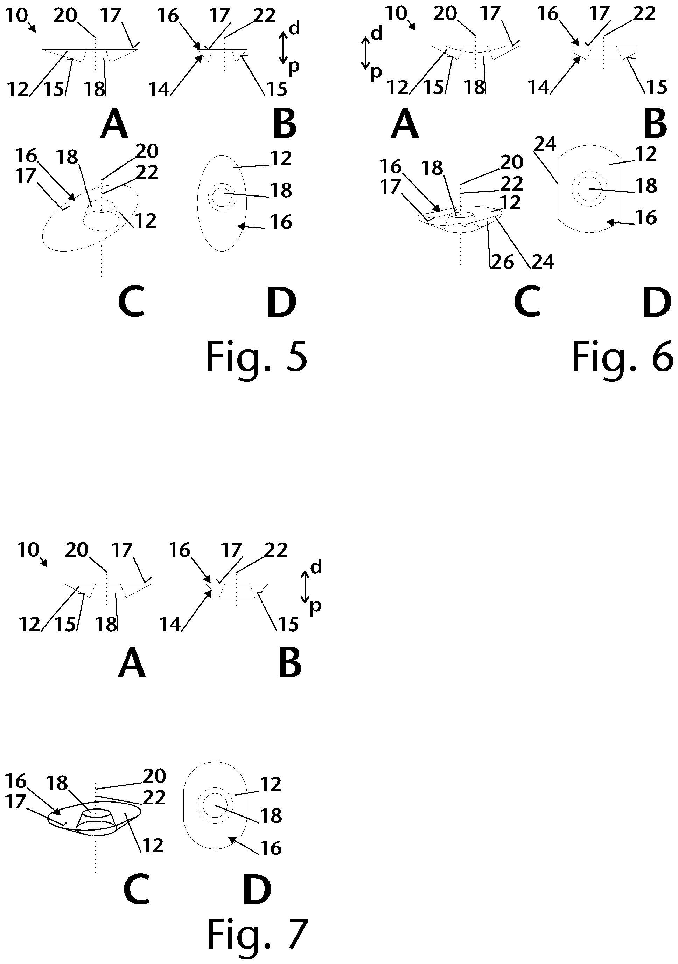

Die konische Ausnehmung 18 ist bevorzugt in Form eines Kreiskegels ausgestaltet. Der Epithesenkörper 12 hingegen weist unterschiedliche Formen der Grundfläche beziehungsweise der distalen Oberfläche 17 auf und kann wie in den Fig. 1 bis 7 gezeigt rechteckig, oval, kreisrund oder jede andere beliebige Form aufweisen. So zeigt beispielsweise Fig. 1 eine rechteckige distale Oberfläche 17, deren nicht weiter bezeichnete Ecken abgerundet sind. Fig. 2 zeigt eine Variante des Epithesenkörpers 12, dessen distale Oberfläche 17 rechteckig ausgestaltet ist, wobei die hier nicht weiter bezeichneten Ecken einen größeren Radius aufweisen als in Fig. 1. Fig. 3 zeigt eine Ausgestaltung, dessen distale Oberfläche 17 im Wesentlichen die Form von zwei vereinigten Kreissegmenten aufweist. Fig. 4 zeigt eine kreisförmige distale Oberfläche 17, die somit einen kreiskegelförmigen Epithesenkörper 12 bildet. Fig. 5 zeigt eine ovale Ausgestaltung der distalen Oberfläche 17, bei der insbesondere die Längsachse 20 Ausnehmung 18 und die Hochachse 22 des Epithesenkörpers 12 im Wesentlichen in einem Brennpunkt der Ellipse angeordnet. Fig. 6 zeigt eine Ausgestaltung, die eine kreisrunde Grundform aufweist, wobei diese von zwei parallelen Sekanten 24 geschnitten ist. Die Tracheostomaepithese aus Fig. 6 weist des Weiteren die Besonderheit auf, dass die Kegelform und den geschnitten Sekanten 24 durch eine Schnittfläche 26 unterbrochen ist. Fig. 7 zeigt eine Ausgestaltung der distalen Seite 16 im Wesentlichen als Ellipse, wobei sich die Ausnehmung 18 zentral durch den Epithesenkörper 12 erstreckt.

Bevorzugt ist vorgesehen, dass der Epithesenkörper 12 ein Epithesenmaterial aufweist, das in im allgemeinen Beschreibungsteil erläutert ist. Auch weitere oben beschriebene Ausführungen können mit den in den Figuren beschriebenen Ausführungen kombiniert werden. Insbesondere die im allgemeinen Beschreibungsteil beschriebenen Ausführungen zur konischen Ausnehmung 18 können auch für die in den Figuren gezeigten Ausgestaltungen gelten.

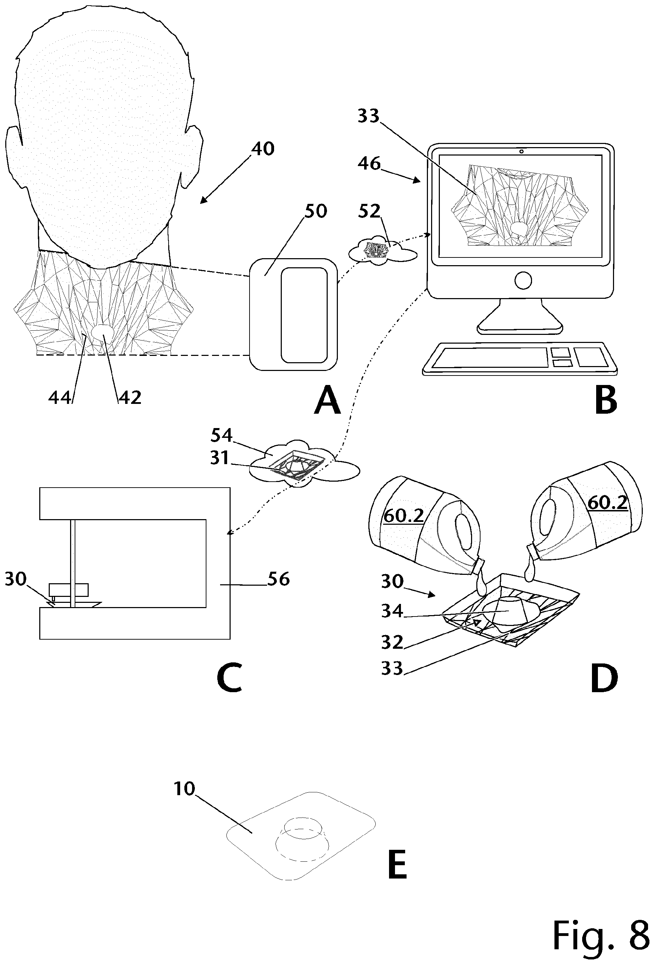

In einer Ausgestaltung ist vorgesehen, dass die proximale Oberfläche 15 der Ausgestaltungen von Fig. 1 bis Fig. 7 einer in Fig. 8 gezeigten Epithesenaufnahmeoberfläche 44, wie im allgemeinen Beschreibungsteil bereits erläutert, angepasst ist.

Fig. 8 zeigt ein Verfahren zur Herstellung einer Tracheostomaepithese 10, wobei die Tracheostomaepithese 10 nicht vorkonfektioniert sondern individuell an die Epithesenaufnahmeoberfläche 44 eines Benutzers 40 angepasst wird. Ein 3D-Scanner 50 scannt die Epithesenaufnahmeoberfläche 44 um das Tracheostoma 42 eines Benutzers 40 und sendet die Scandaten 52 an einen Rechner 46. Mittels des Rechners 46 werden die Scandaten automatisch, halbautomatisch oder manuell bearbeitet und ein Strukturabbild 33 erzeugt, dass in eine virtuelle Epithesen-Gussform 31 eingefügt wird. Aus der virtuellen Epithesen-Gussform 31 werden 3D-Druckerdaten 54 generiert. Die 3D-Druckerdaten 54 werden an einen 3D-Drucker 56 gesendet, der mittels eines generativen Fertigungsverfahrens eine Epithesen-Gussform 30 herstellt. Entweder wird ein Kern 34 unmittelbar beim Drucken in die Epithesen-Gussform 30 eingefügt oder dieser wird nachträglich in die Epithesen-Gussform 30 eingesetzt. Die Epithesen-Gussform 30 bildet eine Kavität 32, in welche Epithesenmaterial 60.1 und 60.2, das insbesondere als 2-Komponenten-Silikonkautschuk ausgestaltet ist, eingebracht wird. Besonders bevorzugt wird das Epithesenmaterial 60.1 und 60.2 in der Epithesen-Gussform 30 in einem Ofen ausgehärtet und danach die fertige Tracheostomaepithese 10 aus der Epithesen-Gussform 30 entformt. In einer besonders bevorzugten Ausgestaltung ist vorgesehen, dass die Epithesen-Gussform 30 mehrfach verwendbar ist, so dass dem Benutzer 40 mittels dieser immer wieder eine neue Tracheostomaepithese 10 anfertigbar ist.

Die vorgeschlagene Tracheostomaepithese 10 ermöglicht einen vorteilhaften Luftstrom in und aus dem Tracheostoma und hat weiterhin den Vorteil, dass ein Tracheostomahilfsmittel wie beispielsweise ein Tracheostmapflaster komfortabel auf der Tracheostomaepithese 10 und dem umliegenden Hautgewebe des Benutzers 40 aufbringbar ist. Die konische Ausnahme bringt weiterhin den Vorteil, dass Tracheostomahilfsmittel, wie beispielsweise Trachealkanülen, gut im Tracheostoma sitzen und abgedichtet sind und dennoch leicht auswechselbar sind, insbesondere ohne die Tracheostomaepithese zu entfernen. Insbesondere kann mit entsprechenden Tracheostomahilfsmitteln eine Sprechfunktion gewährleistet werden und das Tracheostoma umliegende Gewebe wird vorteilhaft geschützt.

## Patentansprüche

1. Tracheostomaepithese (10) umfassend einen Epithesenkörper (12) mit einer proximalen, das heißt zum Patienten hin angeordneten, Seite (14), einer distalen, das heißt vom Patienten weg angeordneten, Seite (16) und einer vollständig von proximal (p) nach distal (d) durchgehenden Ausnehmung (18), wobei die Ausnehmung (18) im Wesentlichen konisch ist, **dadurch gekennzeichnet, dass** sich die Ausnehmung (18) nach distal (d) verjüngt.

2. Tracheostomaepithese (10) gemäß Anspruch 1, **dadurch gekennzeichnet, dass** der Epithesenkörper (12) im Wesentlichen kegelförmig ist, wobei eine distale Oberfläche (17) eine Grundfläche einer Kegelform bildet.

3. Tracheostomaepithese (10) nach einem oder mehreren der vorhergehenden Ansprüche, **dadurch gekennzeichnet, dass** diese zumindest ein Epithesenmaterial (60.1, 60.2) aufweist ausgewählt aus einer Gruppe umfassend Silikonkautschuk, Silikonelastomere und/oder thermoplastische Elastomere.

4. Epithesen-Gussform (30) zur Herstellung eine Tracheostomaepithese (10) zumindest umfassend eine Kavität (32), **dadurch gekennzeichnet, dass** diese einen konischen Kern (34) umfasst, wobei der konische Kern (34) derart in der Kavität (32) angeordnet ist, dass eine Grundfläche des Konus (34) einem proximalen Teil der Gussform (30) zugeordnet ist, der die proximale, das heißt zum Patienten hin angeordneten, Seite (14) der Tracheostomaepithese (10) formt.

5. Epithesen-Gussform (30) gemäß Anspruch 4, **dadurch gekennzeichnet, dass** diese mittels eines generativen Fertigungsverfahrens oder eines Tiefziehverfahrens hergestellt ist.

6. Epithesen-Gussform (30) gemäß einem oder mehreren der Ansprüche 4 bis 5, **dadurch gekennzeichnet, dass** diese zumindest ein Gussformmaterial ausgesucht aus einer Gruppe umfassend bioverträgliche Kunststoffe und/oder Photopolymere umfasst.

7. Verwendung einer Epithesen-Gussform (30) gemäß einem oder mehreren der Ansprüche 4 bis 6 als Verpackungseinleger.

8. Verfahren zur Herstellung einer Tracheostomaepithese (10), umfassend die Schritte
- Bereitstellen einer Epithesen-Gussform (30),
- Einbringen eines konischen Kerns in die Epithesen-Gussform (30),
- Gießen eines Epithesenmaterials in die Epithesen-Gussform (30).

9. Verfahren zur Herstellung einer Tracheostomaepithese (10) gemäß Anspruch 8, **dadurch gekennzeichnet, dass** das Bereitstellen der Gussform (30) die Schritte umfasst
- Abscannen einer Epithesenaufnahmeoberfläche (44) mittels eines 3D-Scanners (50),
- Nacharbeiten von Scandaten (52) und generieren eines Strukturabbilds (33) der Epithesenaufnahmeoberfläche (44) und generieren von 3D-Druckerdaten (54) einer virtuellen Epithesen-Gussform (31) mit diesem Strukturabbild (33),
- Drucken der Epithesen-Gussform (30) entsprechend der 3D-Druckerdaten (54) mittels eines generativen Fertigungsverfahrens.

## Claims

1. Tracheostoma epithesis (10) comprising an epithesis body (12) having a proximal, i.e. patient facing, side (14), a distal side (16), i.e. facing away from the patient, and a recess (18) extending completely from proximal (p) to distal (d), the recess (18) being substantially conical, **characterized in that** the recess tapers towards distal (d).

2. Tracheostomy epithesis (10) according to claim 1, **characterized in that** the epithesis body (12) is substantially conical, wherein a distal surface (17) forms a base of a cone shape.

3. Tracheostomy epithesis (10) according to one or more of the preceding claims, **characterized in that** it comprises at least one epithesis material (60.1, 60.2) selected from a group comprising silicone rubber, silicone elastomers and/or thermoplastic elastomers.

4. Epithesis mould (30) for manufacturing a tracheostomy epithesis (10) comprising at least one cavity (32), **characterized in that** it comprises a conical core (34), said conical core (34) being arranged in said cavity (32) such that a base of said cone (34) is associated with a proximal portion of said mould (30) forming the proximal, i.e. patient facing, side (14) of said tracheostomy epithesis (10).

5. Epithesis mould (30) according to claim 4, **characterized in that** it is produced by means of a generative manufacturing process or a deep-drawing process.

6. Epithesis mould (30) according to one or more of claims 4 to 5, **characterized in that** it comprises at least one mould material selected from a group comprising biocompatible plastics and/or photopolymers.

7. Use of an epithesis mould (30) according to one or more of claims 4 to 6 as a packaging insert.

8. Method of manufacturing a tracheostomy epithesis (10), comprising the steps of
- providing an epithesis mould (30),
- placing a conical core into the epithesis mould (30),
- casting an epithesis material into the epithesis mould (30).

9. Method for manufacturing a tracheostomy epithesis (10) according to claim 8, **characterized in that** providing the mould (30) comprises the steps
- scanning an epithesis receiving surface (44) using a 3D scanner (50),
- post-processing scan data (52) and generating a structural image (33) of the epithesis receiving surface (44) and generating 3D printer data (54) of a virtual epithesis mould (31) with this structural image (33),
- printing the epithesis mould (30) according to the 3D printer data (54) by means of a generative manufacturing process.

## Revendications

1. Epithèse de trachéotomie (10) comprenant un corps d'épithèse (12) ayant une face proximale (14), c'est-à-dire disposée vers le patient, une face distale (16), c'est-à-dire disposée à distance du patient, et un évidement (18) s'étendant complètement du proximal (p) au distal (d), le dit évidement (18) étant sensiblement conique, **caractérisée en ce que** le dit évidement (18) se rétrécit vers ledit distal (d).

2. Epithèse de trachéotomie (10) selon la revendication 1, **caractérisée en ce que** ledit corps d'épithèse (12) est sensiblement conique, une surface distale (17) formant une base en forme de cône.

3. Epithèse de trachéotomie (10) selon une ou plusieurs des revendications précédentes, **caractérisée en ce qu'**elle comprend au moins un matériau d'épithèse (60.1, 60.2) choisi dans un groupe comprenant du caoutchouc de silicone, des élastomères de silicone et/ou des élastomères thermoplastiques.

4. Moule d'épithèse (30) pour produire une épithèse de trachéotomie (10) comprenant au moins une cavité (32), **caractérisé en ce qu'**il comprend un noyau conique (34), ledit noyau conique (34) étant disposé dans ladite cavité (32) de telle sorte qu'une base dudit cône (34) est associée à une partie proximale dudit moule (30) qui forme ladite face proximale (14), c'est-à-dire disposée vers le patient, de ladite épithèse de trachéotomie (10).

5. Moule d'épithèse (30) selon la revendication 4, **caractérisé en ce qu'**il est produit au moyen d'un procédé de production générative ou d'un procédé d'emboutissage profond.

6. Moule d'épithèse (30) selon une ou plusieurs des revendications 4 à 5, **caractérisé en ce qu'**il comprend au moins un matériau du moule choisi dans un groupe comprenant des plastiques biocompatibles et/ou des photopolymères.

7. Utilisation d'un moule d'épithèse (30) selon une ou plusieurs des revendications 4 à 6 comme insert d'emballage.

8. Procédé de production d'une épithèse de trachéotomie (10) comprenant les étapes suivantes
- Fournir un moule d'épithèse (30),
- Introduire un noyau conique dans ledit moule d'épithèse (30),
- Verser un matériau d'épithèse dans ledit moule d'épithèse (30).

9. Procédé de production d'une épithèse de trachéotomie (10) selon la revendication 8, **caractérisé en ce que** la mise à disposition dudit moule (30) comprend les étapes suivantes
- Scanner une surface réceptrice (44) d'épithèse à l'aide d'un scanner 3D (50),
- Travailler des données du scannage (52) et générer une image structurelle (33) de ladite surface réceptrice (44) d'épithèse et générer des données d'imprimante 3D (54) d'un moule d'épithèse virtuel (31) ayant ladite image structurelle (33),
- Imprimer ledit moule d'épithèse (30) selon lesdites données d'imprimante 3D (54) en utilisant un procédé de production générative.
